# FASCICULE DE BREVET EUROPEEN

(11) **EP 0 538 250 B1**
(45) Date de publication et mention de la délivrance du brevet: **07.06.1995**
(21) Numéro de dépôt: 90910768.2
(22) Date de dépôt: 29.06.1990
(51) Int. Cl.: A61K 6/00, A61C 19/10

(54) **PROCEDE DE COLORATION DE PROTHESES MEDICALES ET DISPOSITIF POUR SA MISE EN UVRE**
VERFAHREN ZUM FÄRBEN VON MEDIZINISCHEN PROTHESEN UND VORRICHTUNG ZUR DURCHFÜHRUNG DIESES VERFAHRENS
METHOD FOR DYEING MEDICAL PROSTHESIS AND DEVICE FOR IMPLEMENTING SAME

(43) Date de publication de la demande: 28.04.1993
(73) Titulaire: Duret, François, F-38690 Le-Grand-Lemps (FR)
(72) Inventeur: Duret, François, F-38690 Le-Grand-Lemps (FR)
(74) Mandataire: Maureau, Philippe
(86) Numéro de dépôt international: FR9000489
(87) Numéro de publication internationale: WO9200056

(56) Documents cités:
- DE-A- 3 731 492
- FR-A- 2 642 959
- GB-A- 2 000 174

## Description

La présente invention a pour objet un procédé de coloration de prothèses médicales et un dispositif pour sa mise en oeuvre.

Le document US-A-4 570 204 et le document Television Engineering Handbook (1986), K. Blair Benson, Mc Graw-Hill Book Company, pages 12.24-12.25, décrivent des dispositifs comprenant plusieurs sources d'émission de faisceaux et un système de focalisation de position réglable.

Il est fréquent, voire obligatoire, dans le domaine des prothèses médicales, et notamment des prothèses dentaires, fixes ou mobiles, quel que soit leur mode d'obtention, de réaliser une coloration pour les assortir à leur environnement.

Dans le cas d'une couronne dentaire, par exemple, il est connu d'utiliser des résines renfermant des pigments plus ou moins foncés et dont le choix est effectué en tenant compte de la couleur des dents voisines à l'aide d'un nuancier de références.

A chaque nuance correspond une poudre de céramique ou une résine, l'obtention de la couleur définitive résultant d'un choix judicieux de ces colorants.

Il est également connu, lorsque l'on utilise une céramique vitreuse sans couleur, de réaliser un maquillage de surface ou un maquillage par transparence. Il s'agit, en quelque sorte, de peindre la surface de la dent.

Pour obtenir un aspect de profondeur de la couleur, il est, par ailleurs, connu que le céramiste utilise, en plus des poudres de coloration, des couches successives d'épaisseur transparente disposées entre le colorant et la surface de la dent. Ainsi, pour réaliser une prothèse céramométallique, le praticien doit placer successivement une couche de métal, une couche de recouvrement de celui-ci qui est opaque, une couche de colorant, une couche de matériau transparent et une couche de vernis superficiel.

Il est ainsi possible, dans certaines prothèses, d'avoir sept couches successives.

La présente invention vise à remédier à ces inconvénients en fournissant un procédé de coloration de conception simple, de mise en oeuvre rapide et donnant d'excellents résultats, permettant de donner à une prothèse et notamment à une prothèse dentaire, une couleur en harmonie avec son environnement.

A cet effet, le procédé qu'elle concerne consiste à inclure, dans la masse de la prothèse ou à la surface de celle-ci, au moins un type de pigments photo- ou thermo-chromiques, puis à projeter sur la prothèse un faisceau focalisé ou non, de nature adaptée à celle des pigments de façon à faire réagir ceux-ci.

Le faisceau projeté peut être un faisceau lumineux, calorique ou d'un autre type de rayonnement tel que rayons X.

Cette technique permet de n'avoir qu'un seul matériau prothétique ou de reconstitution mécanique, intégrant le système de coloration et de créer l'effet des couleurs a postériori après fabrication de la pièce.

Cette technique permet de rendre la couleur naturelle en tout point de la matière en jouant sur la focalisation ou sur la défocalisation du faisceau, ce qui évite les constructions en couches successives. Cet aspect naturel de la coloration résulte notamment de la progressivité de la défocalisation du faisceau naturellement au bord du chant, ce qui évite les ruptures brutales.

Selon une premier mode de mise en oeuvre, chaque type de pigments est de coloration irréversible.

Selon un autre mode de mise en oeuvre, chaque type de pigments est de coloration réversible, ce qui permet, lorsque la teinte obtenue après émission d'un ou plusieurs faisceaux, n'est pas satisfaisante, de revenir à l'état initial par émission d'un nouveau faisceau.

Selon un mode de mise en oeuvre, ce procédé consiste à réaliser une opération de fixation de la coloration des pigments. Cette fixation de la coloration des pigments peut être obtenue par envoi d'un faisceau sur la prothèse, par une méthode électrique telle que ionophorèse ou électrophorèse, ou encore par réaction chimique après dépôt d'une substance de fixation sur la surface de la prothèse.

Cette fixation peut avoir lieu par libération de radicaux ou en rendant actives des molécules capables de se fixer sur les pigments et d'empêcher ceux-ci de revenir à leur état initial, ce qui assure le maintien de la coloration.

Le faisceau projeté sur la prothèse pour faire réagir les pigments peut être un faisceau de lumière non cohérente, ce faisceau passant à travers des filtres, par exemple montés sur un barillet, ces filtres étant de couleur correspondant aux bandes d'absorption de chaque pigment.

Il est également possible de projeter, sur la prothèse, au moins un faisceau laser de type laser filtré, utilisant une bande particulièrement active pour chaque pigment, ou un faisceau laser solide dont on choisit la bande active pour chaque pigment à faire réagir.

Il est possible de projeter sur la prothèse, simultanément ou successivement, plusieurs faisceaux dont chacun est destiné à faire réagir un type de pigments. Comme indiqué précédemment, il est possible, au cours de la projection d'un faisceau, de faire varier la focalisation de celui-ci pour obtenir l'effet esthétique souhaité.

A titre d'exemple, une teinte globale brune sera obtenue par un faisceau très absorbé par des pigments photo-chrome, comme le sont par exemple les halogénures d'argent. En jouant sur le temps de latence de retour à l'origine, il est possible de contrôler la couleur obtenue. D'autres pigments réagissant en donnant des couleurs bleues permettent d'obtenir un effet de transparence particulièrement intéressant dans le cas de prothèses dentaires, notamment dans les zones correspondant aux bords des dents.

Il est à noter qu'il est possible, à l'aide de plusieurs types de pigments rouges, verts et bleus, mis en oeuvre simultanément, d'obtenir une infinité de nuances en faisant réagir plus ou moins ces pigments.

Un dispositif convenant à la mise en oeuvre de ce procédé comprend un calculateur connaissant la forme générale de la prothèse à colorer, la nature des pigments et la réactivité de ceux-ci, les sources d'émission de faisceaux, les latitudes de mouvement du système de focalisation, et un dispositif d'analyse de la couleur de la prothèse, le calculateur commandant, à partir de l'information de la teinte à obtenir et en suivant l'opération de coloration, la sélection d'émission des faisceaux des différentes sources, le contrôle de la focalisation, et la fixation de la teinte lorsque la nuance souhaitée est atteinte.

Avantageusement, ce dispositif comprend au moins une source d'émission d'un faisceau, et un système optique mobile de focalisation de position réglable qui assure, successivement, la déviation du faisceau émis par chaque source vers le système de focalisation.

Selon une possibilité, la source d'émission est montée à la place de la broche porte-outil d'une machine-outil à commande numérique, la prothèse étant, pour sa part, fixée sur un support d'usinage. Il est également possible de mettre à profit la broche porte-outil d'une machine-outil en fixant sur celle-ci une tête d'émission qui est reliée par une fibre optique à la source d'émission.

De toute façon, l'invention sera bien comprise à l'aide de la description qui suit, en référence au dessin schématique annexé représentant, à titre d'exemples non limitatifs, plusieurs formes d'exécution d'un dispositif pour la mise en oeuvre de ce procédé :
Figure 1 est une vue d'un schéma bloc représentatif d'un dispositif ;
Figure 2 est une vue d'un premier système optique ;
Figures 3 et 4 sont deux vues représentant deux systèmes d'émission d'un faisceau lumineux ;
Figure 5 est une vue schématique représentant une opération de défocalisation en cours d'émission ;
Figure 6 est une vue d'un montage d'une source d'émission sur un robot trois axes ;
Figures 7 et 8 sont deux vues décrivant le montage d'une tête d'émission sur le porte-outil d'une machine outil ;
Figure 9 est une vue d'un exemple de réaction d'un pigment.

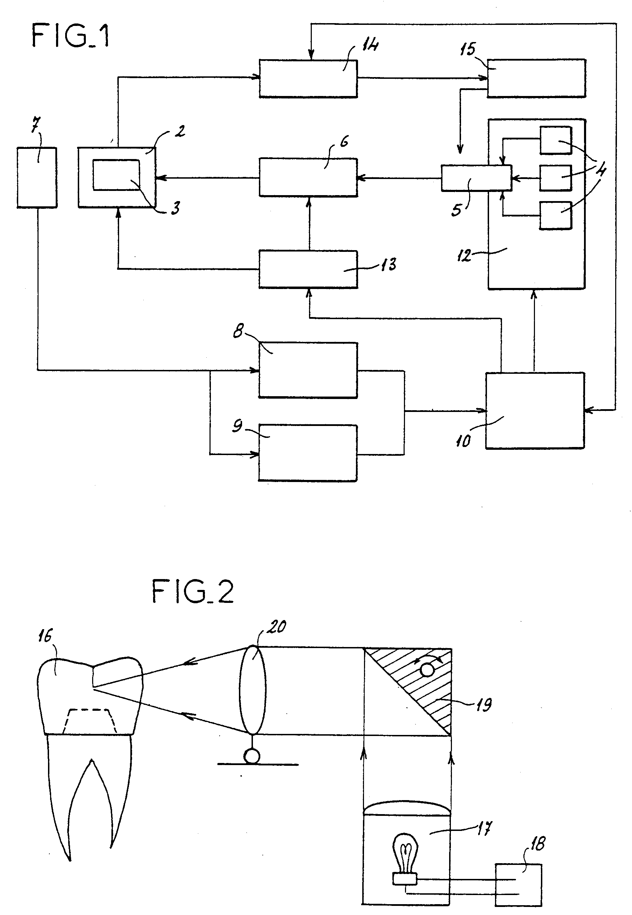

La figure 1 représente un dispositif de coloration d'une prothèse 2 dans la masse ou sur la surface de laquelle se trouvent des pigments 3 photosensibles. L'émission de lumière est réalisée à partir de plusieurs sources 4 spécifiques à chaque pigment à colorer, avec interposition d'un système de sélection de source 5 et d'un système de focalisation 6.

Ce dispositif comprend un système 7 d'analyse de la couleur des dents voisines de la prothèse, s'il s'agit d'une prothèse dentaire, la couleur des dents voisines constituant la couleur de référence à obtenir.

Ce dispositif d'analyse est relié à un bloc 8, 9 de calcul des couleurs qui donne des informations à un système 10 de géographie des couleurs, commandant, d'une part, le générateur 12 équipé des sources 4 et, d'autre part, le bloc 13 de commande du système de focalisation 6.

Ce dispositif comprend également un système de contrôle 14 agissant sur le bloc 10 de géographie des couleurs pour assurer le suivi de l'émission des différents faisceaux lumineux et sur le bloc 15 sélectionnant un faisceau assurant la fixation des pigments dans la situation dans laquelle ils se trouvent lorsque la couleur obtenue correspond à la couleur de référence.

La figure 2 représente une prothèse dentaire 16 contenant des pigments colorés, sur laquelle une insolation est réalisée à partir d'une source lumineuse 17 équipée d'un variateur de puissance 18, le faisceau émis par la source étant réfléchi par un prisme 19 sur un système de focalisation 20 déplaçable dans le sens du faisceau.

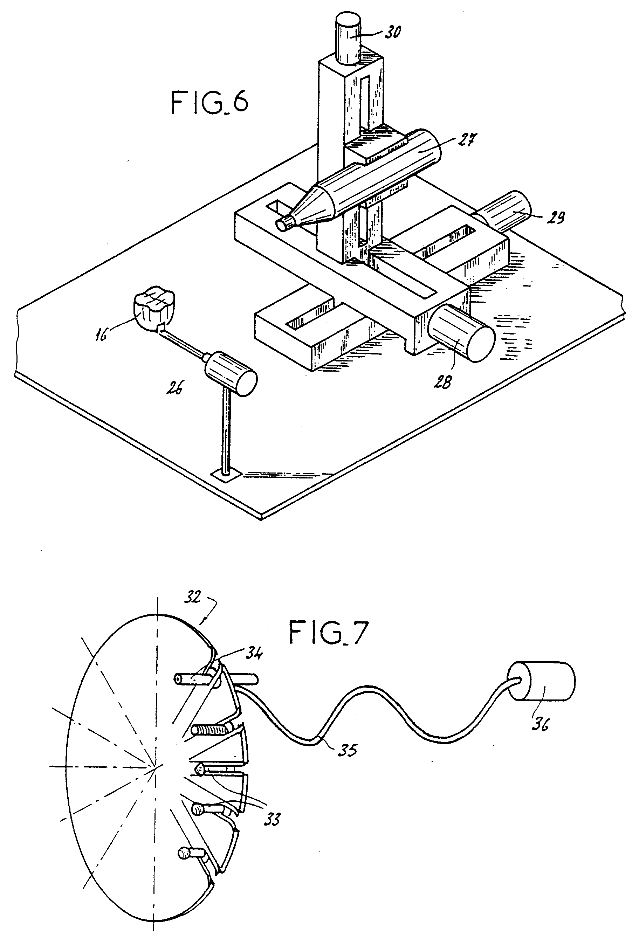

Comme montré à la figure 5, le déplacement du système de focalisation 20 permet de réaliser une défocalisation autorisant un nuancement de la coloration des pigments.

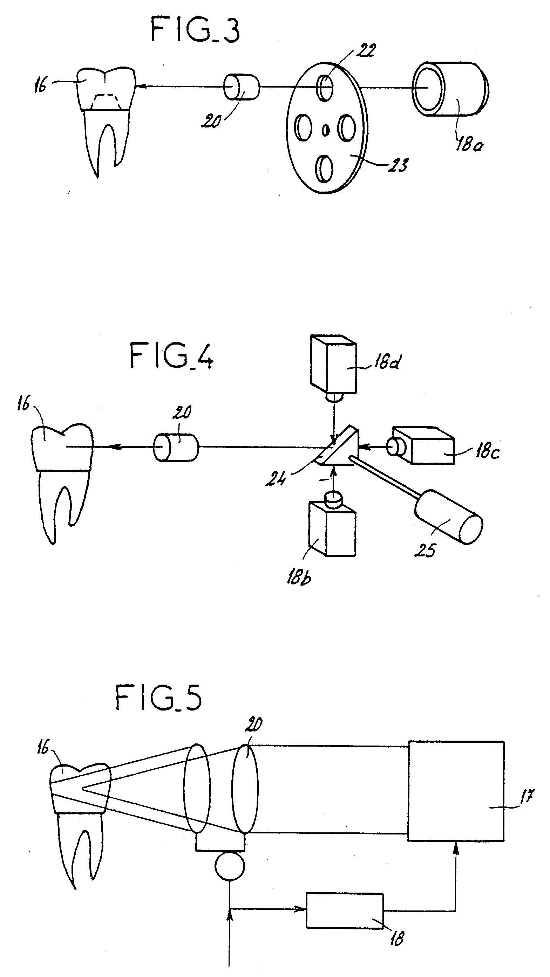

Dans la forme d'exécution représentée à la figure 3, la source 18a est une source de lumière non cohérente émettant un faisceau susceptible de traverser des filtres 22 qui, montés dans un barillet 23, disposé entre la source et le système de focalication, sont de couleur correspondant aux bandes d'absorption de chaque pigment.

Dans la forme d'exécution représentée à la figure 4, plusieurs sources 18b, 18c et 18d, émettent des faisceaux qui sont transmis par l'intermédiaire d'un prisme 24 entraîné en rotation par un moteur 25 sur le système de focalisation 20.

Dans la forme d'exécution, représenteé à la figure 6, la prothèse 16 est montée à une extrémité d'un bras de fixation 26, le faisceau étant constitué par une source laser 27 montée sur un robot trois axes entraîné par des moteurs 28, 29 et 30 dans trois directions croisées.

La figure 7 représente un magasin 32 d'outils 33 utilisés par une machine-outil à commande numérique pour l'usinage d'une prothèse. Dans l'un des logements du disque 32 est placée une tête 34 d'émission d'un faisceau; reliée par une fibre optique 35 à une source 36.

Cette tête 34 peut, de même que les outils 33, être fixée sur la broche porte-outil 37 de la machine outil à commande numérique.

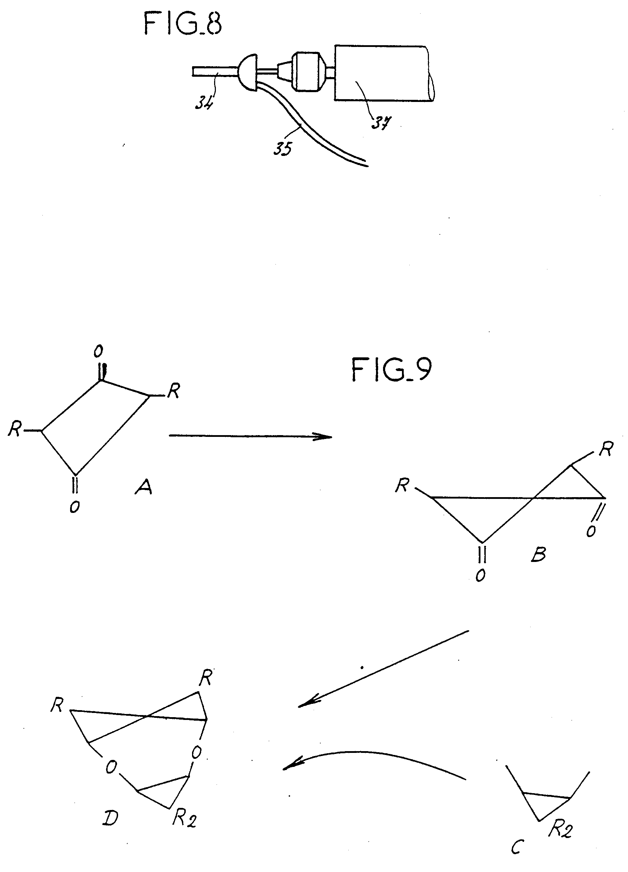

La figure 9 représente, à titre d'exemple, un processus de coloration. Un pigment non coloré se trouvant à l'état A change de couleur sous l'action d'un rayonnement par modification de sa structure moléculaire et se trouve dans un état B.

Sous l'action d'un autre rayonnement, et par association d'une molécule de stabilisation C, le pigment peut être amené dans un etat D où il est à la fois coloré et stable.

Comme il ressort de ce qui précède l'invention apporte une grande amélioration à la technique existante en fournissant un procédé et un dispositif de coloration des prothèses médicales, et notamment de prothèses dentaires, de conception simple, de mise en oeuvre rapide et permettant d'obtenir les nuances de couleurs souhaitées.

Comme il va de soi, l'invention ne se limite pas au seul mode de mise en oeuvre de ce procédé, ni aux seules formes d'exécution de ce dispositif, décrits ci-dessus à titre d'exemples : elle en embrasse, au contraire, toutes les variantes.

C'est ainsi notamment que la modification de la couleur des pigments pourrait être obtenue sous l'action d'un rayonnement calorique, sans que l'on sorte pour autant du cadre de l'invention.

## Revendications

1. Procédé de coloration de prothèses médicales, caractérisé en ce qu'il consiste à inclure dans la masse de la prothèse (16) ou à la surface de celle-ci au moins un type de pigments photo- ou thermo-chromiques, puis à projeter sur la prothèse un faisceau focalisé ou non, de nature adaptée à celle des pigments de façon à faire réagir ceux-ci.

2. Procédé selon la revendication 1, caractérisé en ce que chaque type de pigment mis en oeuvre est de coloration irréversible.

3. Procédé selon la revendication 1, caractérisé en ce que chaque type de pigment mis en oeuvre est de coloration réversible.

4. Procédé selon l'une quelconque des revendications 1 à 3, caractérisé en ce qu'il consiste à réaliser une opération de fixation de la coloration des pigments.

5. Procédé selon la revendication 4, caractérisé en ce que la fixation de la coloration des pigments est réalisée par envoi d'un faisceau sur la prothèse.

6. Procédé selon la revendication 4, caractérisé en ce que la fixation de la coloration des pigments est réalisée par une méthode électrique telle que ionophorèse ou électrophorèse.

7. Procédé selon la revendication 4, caractérisé en ce que la fixation de la coloration des pigments est réalisée par une méthode chimique, par dépôt d'une substance de fixation sur la surface de la prothèse.

8. Procédé selon l'une quelconque des revendications 1 à 7, caractérisé en ce qu'il consiste à réaliser la modification de l'état des pigments par projection sur la prothèse d'au moins un faisceau lumineux.

9. Procédé selon la revendication 8, caractérisé en ce qu'il consiste à projeter sur la prothèse au moins un faisceau laser de type laser filtré, utilisant une bande particulièrement active pour chaque pigment, ou un faisceau laser solide dont on choisit la bande active pour chaque pigment à faire réagir.

10. Procédé selon la revendication 8, caractérisé en ce qu'il consiste à projeter sur la prothèse un faisceau de lumière non cohérente, et à faire passer ce faisceau dans des filtres de couleur correspondant aux bandes d'absorption de chaque pigment.

11. Procédé selon l'une quelconque des revendications 1 à 10, caractérisé en ce qu'il consiste à projeter sur la prothèse, simultanément ou successivement, plusieurs faisceaux dont chacun est destiné à faire réagir un type de pigment.

12. Procédé selon l'une quelconque des revendications 1 à 11, caractérisé en ce qu'il consiste à réaliser au cours de la projection du faisceau sur la prothèse une défocalisation du faisceau.

13. Dispositifs convenant à la mise en oeuvre du procédé selon l'une des revendications 1 à 12, caractérisé en ce qu'il comprend un calculateur connaissant la forme générale de la prothèse à colorer, la nature des pigments et la réactivité de ceux-ci, les sources d'émission de faisceaux, les latitudes de mouvement du système de focalisation, et un dispositif (7) d'analyse de la couleur de la prothèse, le calculateur commandant, à partir de l'information de la teinte à obtenir et en suivant l'opération de coloration, la sélection (5) d'émission des faisceaux de différentes sources, le contrôle de la focalisation, et la fixation (15) de la teinte lorsque la nuance souhaitée est atteinte.

14. Dispositif selon la revendication 13, caractérisé en ce qu'il comprend plusieurs sources d'émission (18b, 18c, 18d) d'un faisceau, et un système optique mobile tel qu'un prisme (24), pour réaliser la déviation du faisceau émis par chaque source vers un système de focalisation (20) de position réglable.

15. Dispositif selon la revendication 14, caractérisé en ce qu'il comprend une source de lumière non cohérente (18a) et un barillet (23) équipé de plusieurs filtres (22), disposé entre la source et le système de focalisation (20).

16. Dispositif selon la revendication 14, caractérisé en ce que la source d'émission (27) est montée à la place de la broche porte-outil d'une machine-outil à commande numérique, la prothèse (16) étant pour sa part fixée sur un support d'usinage (26).

17. Dispositif selon la revendication 14, caractérisé en ce que la source d'émission (36) est fixe et reliée par une fibre optique (35) à une tête d'émission (34) destinée à être stockée sur le magasin d'outils (32) d'une machine-outil, et à être fixée à la broche (37) de la machine-outil préalablement à l'opération d'activation des pigments.

## Claims

1. Method for colouring medical prostheses, characterised in that it consists of including in the mass of the prosthesis (16) or on the surface thereof at least one type of photo- or thermochromic pigment, and then of projecting onto the prosthesis a beam, focused or unfocused, of a type suited to that of the pigments so as to cause the latter to react.

2. Method according to Claim 1, characterised in that the colouring of each type of pigment used is permanent.

3. Method according to Claim 1, characterised in that the colouring of each type of pigment used is non-permanent.

4. Method according to any one of Claims 1 to 3, characterised in that it consists of carrying out an operation of fixing the colouring of the pigments.

5. Method according to Claim 4, characterised in that the fixing of the colouring of the pigments is effected by projecting a beam onto the prosthesis.

6. Method according to Claim 4, characterised in that the fixing of the colouring of the pigments is effected by means of an electrical method such as ionophoresis or electrophoresis.

7. Method according to Claim 4, characterised in that the fixing of the colouring of the pigments is effected by means of a chemical method, by depositing a fixing substance on the surface of the prosthesis.

8. Method according to any one of Claims 1 to 7, characterised in that it consists of bringing about the change in the state of the pigments by projecting at least one light beam onto the prosthesis.

9. Method according to Claim 8, characterised in that it consists of projecting at least one laser beam of the filtered laser type onto the prosthesis, using a band that is particularly active for each pigment, or a solid laser beam whose band is chosen so as to be active for each pigment to be reacted.

10. Method according to Claim 8, characterised in that it consists of projecting a non-coherent beam of light onto the prosthesis and causing this beam to pass through coloured filters corresponding to the absorption bands of each pigment.

11. Method according to any one of Claims 1 to 10, characterised in that it consists of simultaneously or successively projecting several beams, each of which is designed to cause one type of pigment to react, onto the prosthesis.

12. Method according to any one of Claims 1 to 11, characterised in that it consists of effecting, during the projection of the beam onto the prosthesis, a de-focusing of the beam.

13. Device suitable for implementing the method according to one of Claims 1 to 12, characterised in that it comprises a computer which knows the general shape of the prosthesis to be coloured, the nature of the pigments and their reactivity, the emission sources of beams and the range of movement of the focusing system, and a device (7) for analysing the colour of the prosthesis, the computer, on the basis of the desired colour data and by monitoring the colouring operation, controlling the selection (5) of beam emissions from different sources, the monitoring of focusing and the fixing (15) of the colour when the desired shade is attained.

14. Device according to Claim 13, characterised in that it comprises several beam emission sources (18b, 18c, 18d), and a mobile optical system such as a prism (24), for producing the diversion of the beam emitted by each source towards a focusing system (20) whose position is adjustable.

15. Device according to Claim 14, characterised in that it comprises a non-coherent light source (18a) and a turret (23), equipped with several filters (22) disposed between the source and the focusing system (20).

16. Device according to Claim 14, characterised in that the emission source (27) is mounted in place of the tool-holder spindle of a numerically controlled machine tool, the prosthesis (16) being, for its part, fixed to a machining support (26).

17. Device according to Claim 14, characterised in that the emission source (36) is fixed and connected by an optical fibre (35) to an emission head (34) designed to be stored on the tool magazine (32) of a machine tool, and to be fixed to the spindle (37) of the machine tool prior to the pigment activation operation.

## Patentansprüche

1. Verfahren zum Färben medizinischer Prothesen, **dadurch gekennzeichnet,** daß es darin besteht, in die Masse der Prothese (16) oder der Oberfläche derselben wenigstens eine Art photo- oder thermochromer Pigmente beizufügen und dann auf die Prothese ein fokussiertes oder nichtfokussiertes Bündel zu projizieren, dessen Beschaffenheit auf jene der Pigmente derart abgestimmt ist, daß es diese reagieren läßt.

2. Verfahren nach Anspruch 1, **dadurch gekennzeichnet,** daß jede eingesetzte Art von Pigment von irreversibler Färbung ist.

3. Verfahren nach Anspruch 1, **dadurch gekennzeichnet,** daß jede eingesetzte Art von Pigment von reversibler Färbung ist.

4. Verfahren nach einem der Ansprüche 1 bis 3, **dadurch gekennzeichnet,** daß es darin besteht, einen Vorgang zur Fixierung der Färbung der Pigmente durchzuführen.

5. Verfahren nach Anspruch 4, **dadurch gekennzeichnet**, daß die Fixierung der Färbung der Pigmente mittels eines Bündels auf der Prothese durchgeführt wird.

6. Verfahren nach Anspruch 4, **dadurch gekennzeichnet,** daß die Fixierung der Färbung der Pigmente mittels eines elektrischen Verfahrens, wie Ionophorese oder Elektrophorese, durchgeführt wird.

7. Verfahren nach Anspruch 4, **dadurch gekennzeichnet,** daß die Fixierung der Färbung der Pigmente mittels eines chemischen Verfahrens durch Ablagerung einer Fixierungssubstanz auf der Oberfläche der Prothese durchgeführt wird.

8. Verfahren nach einem der Ansprüche 1 bis 7, **dadurch gekennzeichnet,** daß es darin besteht, die Änderung des Zustands der Pigmente durch Projektion wenigstens eines Lichtbündels auf die Prothese durchzuführen.

9. Verfahren nach Anspruch 8, **dadurch gekennzeichnet,** daß es darin besteht, auf die Prothese wenigstens ein Laserbündel des gefilterten Lasertyps unter Verwendung eines besonders aktiven Bands für jedes Pigment oder ein Bündel eines Feststofflasers zu projizieren, dessen aktives Band man für jedes Pigment, das man reagieren lassen möchte, wählt.

10. Verfahren nach Anspruch 8, **dadurch gekennzeichnet,** daß es darin besteht, auf die Prothese ein nicht-kohärentes Lichtbündel zu projizieren und dieses Bündel Farbfilter durchsetzen zu lassen, deren Farben den Absorptionsbändern jedes Pigments entsprechen.

11. Verfahren nach einem der Ansprüche 1 bis 10, **dadurch gekennzeichnet,** daß es darin besteht, auf die Prothese gleichzeitig oder aufeinanderfolgend mehrere Bündel zu projizieren, von denen jedes dazu bestimmt ist, eine Art von Pigment reagieren zu lassen.

12. Verfahren nach einem der Ansprüche 1 bis 11, **dadurch gekennzeichnet,** daß es darin besteht, im Verlauf der Projektion des Bündels auf die Prothese eine Defokussierung des Bündels durchzuführen.

13. Vorrichtung, geeignet zur Durchführung des Verfahrens nach einem der Ansprüche 1 bis 12, **dadurch gekennzeichnet,** daß sie einen Rechner, der die allgemeine Form der zu färbenden Prothese, die Beschaffenheit der Pigmente und deren Reaktionsvermögen, die Quellen zur Emission von Bündein, die Bewegungsmöglichkeiten des Fokussierungssystems kennt, und eine Vorrichtung (7) zur Analyse der Farbe der Prothese umfaßt, wobei der Rechner ausgehend von der Information des zu erhaltenden und sich im Verlaufe des Färbungsvorgangs ergebenden Farbtons die Auswahl (5) zur Emission der Bündel verschiedener Quellen, die Fokussierungssteuerung und bei Erreichen der gewünschten Nuance die Fixierung (15) des Farbtons steuert.

14. Vorrichtung nach Anspruch 13, **dadurch gekennzeichnet,** daß nie mehrere Quellen (18b, 18c, 18d) zur Emission eines Bündels umfaßt, sowie ein bewegliches optisches System, wie ein Prisma (24), um die Ablenkung des von jeder Quelle emittierten Bündels zu einem Fokussierungssystem (20) steuerbarer Stellung durchzuführen.

15. Vorrichtung nach Anspruch 14, **dadurch gekennzeichnet,** daß sie eine Quelle für nicht-kohärentes Licht (18a) und eine Lochscheibe (23) umfaßt, welche mit mehreren Filtern (22) ausgestattet und zwischen der Quelle und dem Fokussierungssystem (20) angeordnet ist.

16. Vorrichtung nach Anspruch 14, **dadurch gekennzeichnet**, daß die Emissionsquelle (27) am Ort der Werkzeughalterspindel einer numerisch gesteuerten Werkzeugmaschine angebracht ist, wobei die Prothese (16) ihrerseits auf einem Werkstückhalter (26) befestigt ist.

17. Vorrichtung nach Anspruch 14, **dadurch gekennzeichnet,** daß die Emissionsquelle (36) fest und über eine optische Faser (35) mit einem Emissionskopf (34) Verbunden ist, der zur Anbringung auf dem Werkzeugmagazin (32) einer Werkzeugmaschine und zur Befestigung an der Spindel (37) der Werkzeugmaschine vor der Aktivierung der Pigmente bestimmt ist.
